# EUROPÄISCHE PATENTSCHRIFT

(11) **EP 4 042 023 B1**
(45) Veröffentlichungstag und Bekanntmachung des Hinweises auf die Patenterteilung: **03.01.2024**
(21) Anmeldenummer: 20803793.7
(22) Anmeldetag: 06.11.2020
(51) Int. Cl.: F04F 5/00, A61C 17/06

(54) **DENTALABSCHEIDER**
DENTAL SEPARATOR
SÉPARATEUR DENTAL

(30) Priorität: 08.11.2019 AT 509542019
(43) Veröffentlichungstag der Anmeldung: 17.08.2022
(73) Patentinhaber: Pregenzer, Bruno, 6414 Mieming (AT)
(72) Erfinder: Pregenzer, Bruno, 6414 Mieming (AT)
(74) Vertreter: Laufhütte, Dieter
(86) Internationale Anmeldenummer: PCT/EP2020/081274
(87) Internationale Veröffentlichungsnummer: WO 2021/089773

(56) Entgegenhaltungen:
- EP-A1- 2 977 613
- WO-A1-00/71050
- WO-A1-95/12365
- DE-U1-202010 010 802
- FR-A1- 2 622 566
- US-A1- 2018 289 457
- US-B1- 6 276 936

## Beschreibung

Die Erfindung betrifft einen Dentalabscheider für den zahnärztlichen Begandlungsplatz.

Bei einem Dentalabscheider handelt es sich um ein in der Regel kompaktes Bauteil, das an zahnärztlichen Behandlungsplätzen eingesetzt wird, um Feststoffpartikel aus dem Luft-Flüssigkeits-Gemisch abzutrennen, das vom Mund eines Patienten abgesaugt wird.

Eine bekannte Konstruktion eines Dentalabscheiders wird beispielsweise in der WO 2000/071050 A1 vorgestellt. Aus den US 5,018,971 A und US 5,613,851 A sind ebenfalls Beispiele für Dentalabscheider bekannt.

Dentalabscheider mit Strahlpumpen werden in den US 6 276 936 B1, WO 95/12365 A1, EP 2 977 613 A1 und DE 20 2010 010 802 U1 offenbart. Weitere Beispiele für Dentalabscheider, welche allerdings keine Strahlpumpe aufweisen, zeigen die US 2018/289457 A1 und FR 2 622 566 A1.

Aufgabe der Erfindung ist es, einen Dentalabscheider bereitzustellen, der eine verbesserte Trennung zwischen Luft und partikelbeladener Flüssigkeit erreicht.

Vor diesem Hintergrund betrifft die Erfindung einen Dentalabscheider mit einem Gehäuse, in dessen oberen Bereich eine Entwässerungszone zur Abtrennung von partikelhaltiger Flüssigkeit aus einem mit der Flüssigkeit beladenen Luftstrom ausgebildet ist, wobei das Gehäuse einen Einlass für den beladenen Luftstrom aufweist, der tangential in die Entwässerungszone mündet, und im Gehäuse unterhalb der Entwässerungszone eine Abscheidezone zur Abtrennung der Partikel aus der partikelhaltigen Flüssigkeit ausgebildet ist, die von der Entwässerungszone in die Abscheidezone sinkt. Erfindungsgemäß ist vorgesehen, dass die Entwässerungszone eine gekrümmte Strömungsbahn umfasst und der Einlass eine Strahlpumpe mit einem Treibmedienanschluss für ein Treibmedium, einem Saugmedienanschluss für ein Saugmedium und einen Austritt aufweist, wobei der Austritt tangential in die Strömungsbahn mündet.

Die Strahlpumpe umfasst eine Venturi-Düse, in welcher das Treibmedium mit hohem Druck durch eine Verengung getrieben wird, um dort einen Unterdruck zu erzeugen. Aufgrund dieses Unterdrucks wird durch den Saugmedienanschluss, der im Bereich der Verengung in die Düse mündet, ein Saugmedium angesaugt und mit dem Treibmedium vermischt. Die Strahlpumpe zeichnet sich durch ihren einfachen Aufbau und die hohe Betriebssicherheit aus.

In einer bevorzugten Ausführungsvariante ist die Strahlpumpe in der zahnärztlichen Behandlungseinheit als Luftstrahlpumpe verbaut. Bei der Luftstrahlpumpe strömt Druckluft durch die Venturi-Düse. Der dabei entstehende Sog wird für die Absaugung genutzt. Nämlich kann in der zahnärztlichen Behandlungseinheit beispielsweise ein Speichelsauger mit dem Saugmedienanschluss und eine Druckluftquelle mit dem Treibmedienanschluss verbunden sein. Wird der Speichelsauger aus der Lagerungsposition genommen, wird die Druckluftquelle entsichert und die Druckluft strömt durch die Düse, um am Speichelsauger einen Unterdruck zu erzeugen. Das am Speichelsauger angesaugte Luft-Speichel-Gemisch mit dispergierten Festkörperpartikeln wird dann in der Düse mit der Druckluft vermischt und gelangt mit hoher Geschwindigkeit in die ringförmige Strömungsbahn.

An Stelle von Druckluft kann auch ein anderes Fluid wie beispielsweise ein anderes Gas oder auch eine Flüssigkeit wie beispielsweise Wasser als Treibmedium verwendet werden. Bevorzugt ist aber der Einsatz von Luft.

Bei der Druckluftquelle kann es sich beispielsweise um eine unter Druck stehende Gasflasche handeln, wobei ein steuerbares Ventil zwischen der Gasflasche und der Strahlpumpe angeordnet ist, um die Strahlpumpe selektiv aktivieren zu können. Alternativ kann auch eine Pumpe wie beispielsweise eine Luftpumpe zum Einsatz kommen, die selektiv aktiviert werden kann.

Das Gehäuse ist vorzugsweise im Querschnitt rund ausgebildet und verläuft entlang einer in der Einbausituation im Wesentlichen vertikalen Achse. Der Einlass befindet sich oben am Gehäuse. Ein Auslass bzw. Auslässe für den entwässerten Luftstrom bzw. die letztlich partikelfreie Flüssigkeit befinden sich unten am Gehäuse.

Das Gehäuse kann aus mehreren Teilen bestehen. In einer Ausführungsvariante kann das Gehäuse einen unteren Gehäuseteil und einen darauf aufgesetzten oberen Gehäuseteil umfassen, wobei bevorzugt ist, dass das untere Gehäuseteil an seinem Boden geschlossen und nach oben hin offen ist, und dass das obere Gehäuseteil nach oben anhand eines Deckels verschlossen ist und einen Boden aufweist, der den Innenraum des Gehäuses in eine Entwässerungszone und eine Abscheidezone aufteilt. Der Einlass befindet sich vorzugsweise am oberen Gehäuseteil.

Der Einlass mündet vorzugsweise tangential in die Strömungsbahn. In einer Ausführungsvariante kann die Strömungsbahn schneckenförmig um eine fast volle Umdrehung nach innen laufen, bis sie an einem Auslass endet.

Vorzugsweise sind die Prallblättchen gleichmäßig und alle auf derselben Höhe über die Erstreckung des Strömungskanals verteilt und dabei im spitzen Winkel von beispielsweise 10°-40° oder vorzugsweise 20°-30° in Strömungsrichtung nach oben aus der Horizontalen geneigt. Auch eine Krümmung der Prallblättchen in Strömungsrichtung kann vorgesehen sein. Der in der Draufsicht erkennbare Abstand zwischen den Vorderkanten und Hinterkanten aufeinanderfolgender Prallblättchen ist vorzugsweise kleiner als die halbe umfängliche Erstreckung und vorzugsweise kleiner als ein Viertel der umfänglichen Erstreckung der Prallblättchen ist. Weiter vorzugsweise fluchten die Vorderkanten und Hinterkanten aufeinanderfolgender Prallblättchen oder die Prallblättchen überlappen sogar.

Durch eine Ausbildung entsprechend einer oder mehrerer der obenstehend beschriebenen bevorzugten Ausgestaltungen ergibt sich insgesamt eine treppenförmige Kontur der Prallblättchen. Diese Kontur bewirkt, dass der Luftstrom in der Strömungsbahn durch die Prallblättchen kaum verwirbelt wird und dass zudem eine Luftbewegung in der Abscheidezone unterhalb des Prallblättchenkranzes effektiv verhindert wird. So kann eine Agitation der dort gesammelten Flüssigkeit vermieden und eine Sedimentationsabscheidung der in der Flüssigkeit enthaltenen Partikel begünstigt werden. Trotzdem kommt es zu einer effektiven Entwässerung des Luftstroms.

In einer Ausführungsvariante der Erfindung können die Prallblättchen radial von einer gekrümmten Wand innerhalb des Gehäuses abstehen, welche die Innenseite der Strömungsbahn definiert.

In einer Ausführungsform sind an einer Wand, welche die äußere Begrenzung des Strömungskanals bildet und bei der es sich um die Wand eines im Gehäuse eingesetzten Einsatzes oder eine Wand des Gehäusemantels handeln kann, stegartige Leitvorsprünge vorgesehen, die in den Strömungskanal ragen. Die Leitvorsprünge können die Gestalt von im spitzen Winkel von beispielsweise zwischen 5° und 30° nach hinten aus der Vertikalen geneigten und leicht konvex gekrümmten Stegen haben.

Die unteren Enden der Leitvorsprünge können in einer Ausführungsform an der Oberseite eines korrespondierenden Prallblättchens anstehen. Vorzugsweise ist dabei vorgesehen, dass zwischen der Außenkante der Prallblättchen und der Innenoberfläche einer Wand, welche die äußere Begrenzung des Strömungskanals bildet, kann in einer Ausführungsvariante ein Spalt ausgebildet ist. Der Spalt sollte sich in Abflussrichtung, also gegen die Strömungsrichtung an den Berührungspunkt anschließen, sodass am Leitvorsprung abgeschiedenes Wasser nach unten abrinnen kann.

Die Begrenzungswände des Strömungskanals nebst Prallkonturen, also Prallblättchen und gegebenenfalls Leitvorsprünge, können an einem Einsatz ausgebildet sein, der im Gehäuse und vorzugsweise im oberen Gehäuseteil aufgenommen ist. Der Einsatz kann insgesamt als Spritzgussteil gefertigt sein.

Unterhalb des Kranzes an Prallblättchen kann, gegebenenfalls noch im Bereich des oberen Gehäuseteils, eine Auffangzone in Form beispielsweise einer um die Achse des Dentalabscheiders gekrümmte Rinne aufweisen, um das an den Prallkonturen aus dem Luftstrom abgeschiedene Wasser aufzufangen. Aus dieser Auffangzone kann das Wasser durch beispielsweise ein Fallrohr in die Abscheidezone geleitet werden.

Im Bodenbereich des Gehäuses und gegebenenfalls des unteren Gehäuseteils ist eine Sedimentationszone ausgebildet, die nach oben hin anhand eines Filters in Form mindestens eines Siebbodens und gegebenenfalls einer oder mehrerer Schüttungen abgegrenzt ist, wobei die Siebböden und gegebenenfalls Schüttungen Schwebeteilchen aus dem Wasser abreichern. Oberhalb des Filtermediums kann ein Absaugbereich ausgebildet sein.

In einer Ausführungsform weist das Gehäuse ferner einen Auslass für den behandelten Luftstrom auf, der am distalen Ende der gekrümmten Strömungsbahn von der Entwässerungszone nach außen führt. Der Auslass ist entsprechend im oberen Bereich des Gehäuses, beispielsweise am oberen Gehäuseteil angeordnet.

Innerhalb des Auslasses kann eine Venturi-Düse mit einer Verengung ausgebildet sein, in welche ein aus dem Absaugbereich der Abscheidezone führendes Saugrohr mündet. Durch den Luftstrom fungiert der so gestaltete Auslass als Luftstrahlpumpe, anhand derer die gereinigte Flüssigkeit aus der Entwässerungszone gesogen und gemeinsam mit dem zuvor entwässerten Luftstrom nach außen geleitet werden kann.

Die Erfindung betrifft ferner eine Zahnärztliche Behandlungseinheit umfassend einen Speichelsauger, eine Druckquelle und einen erfindungsgemäßen Dentalabscheider, wobei der Speichelsauger mit dem Saugmedienanschluss und eine Druckquelle mit dem Treibmedienanschluss verbunden sind. Bevorzugte Ausgestaltungsmöglichkeiten ergeben sich aus der obenstehenden Diskussion des erfindungsgemäßen Dentalabscheiders.

Weitere Einzelheiten und Vorteile der Erfindung ergeben sich aus dem nachfolgend anhand der Figuren beschriebenen Ausführungsbeispiel. In den Figuren zeigen:

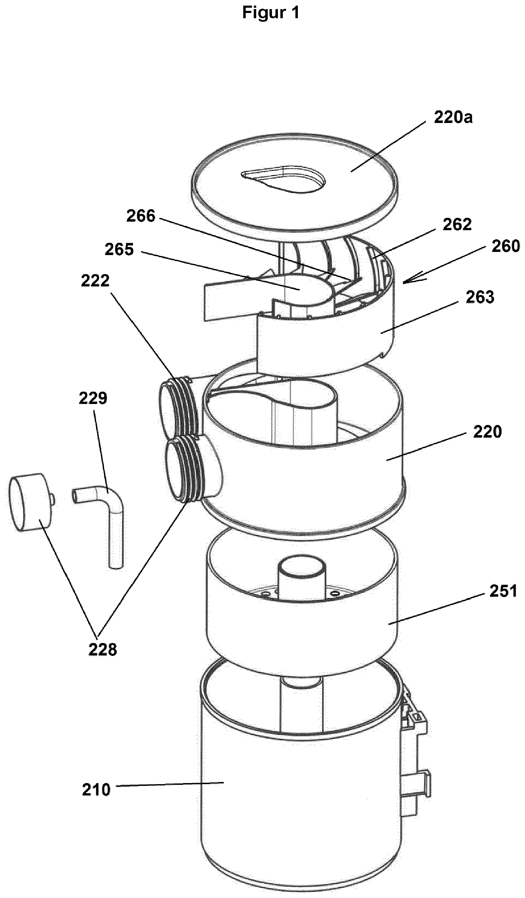

- Figur 1:: eine Explosionsansicht eines Dentalabscheiders gemäß einer Ausführungsform der Erfindung;

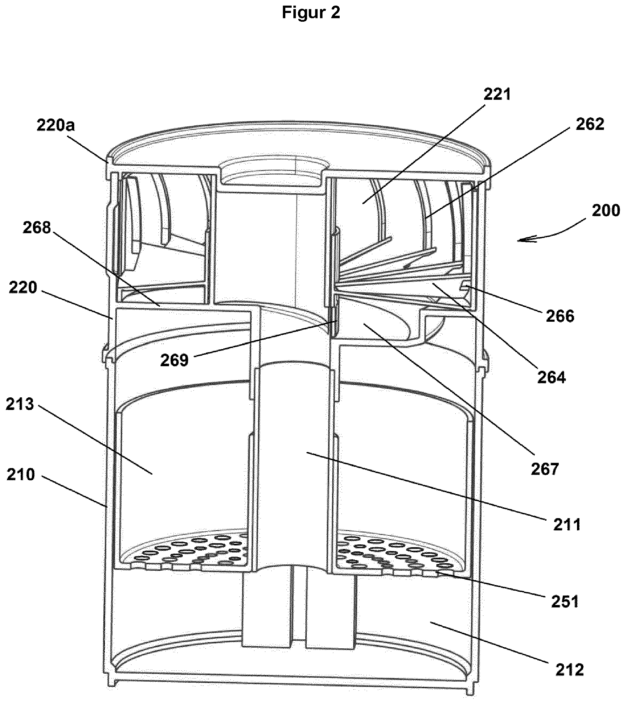

- Figur 2:: einen Längsschnitt durch diesen Dentalabscheider;

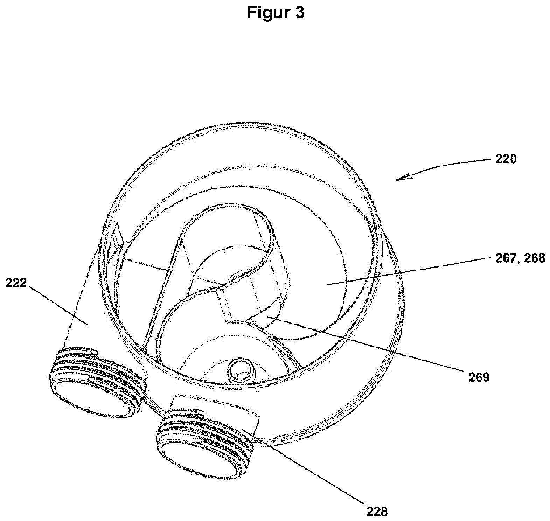

- Figur 3:: eine Ansicht des oberen Gehäuseteils dieses Dentalabscheiders von schräg oben mit abgenommenem Deckel und fehlendem Luftleiteinsatz;

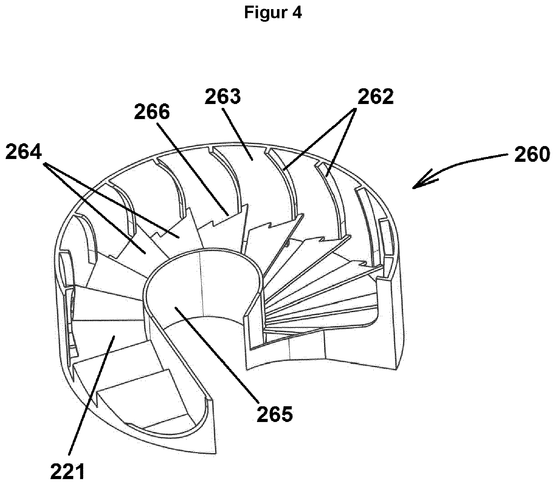

- Figur 4:: eine Ansicht des isolierten Luftleiteinsatzes; und
- Figur 5:: eine Schnittansicht durch den oberen Gehäuseteil des gezeigten erfindungsgemäßen Dentalabscheiders.

Der in den Figuren 9 dargestellte erfindungsgemäße Abscheider 200 umfasst ein im Querschnitt rundes Gehäuse aus Kunststoff, welches sich in diesem Fall aus einem unteren Gehäuseteil 210 und einem oberen Gehäuseteil 220 zusammensetzt. Das untere Gehäuseteil 210 ist an seinem Boden geschlossen und nach oben hin offen. Das obere Gehäuseteil 220 ist ebenfalls nach oben offen, wobei es oben anhand eines Deckels 220a verschlossen ist. Der Boden 268 des oberen Gehäuseteils 220, der den Innenraum des oberen Gehäuseteils 220 vom Innenraum des unteren Gehäuseteils 210 abgrenzt, wird in weiterer Folge noch näher beschreiben.

Das Gehäuse umschließt einen mehrteiligen Innenraum, wobei im Bereich des oberen Gehäuseteils 220 ein Luftleiteinsatz 260 eingesetzt ist, der einen schneckenförmig um eine fast volle Umdrehung nach innen laufenden Entwässerungsraum 221 definiert. Im Bereich des unteren Gehäuseteils 210 schließt sich an den Luftleiteinsatz 260 ein nachfolgend näher beschriebener Sedimentationsbereich an.

Das vom Speichelsauger stammende Luft-Flüssigkeits-Gemisch wird über den am oberen Gehäuseteil 220 angeordneten Einlassstutzen 222 tangential in den Entwässerungsraum 221 eingeleitet und ringsum über eine besondere kaskadenförmige Einheit geführt, die in weiterer Folge noch näher beschrieben werden wird. Dabei wird durch Zentrifugalkräfte und Kollisionen des Luftstroms mit Prallflächen die partikelhaltige Flüssigkeit, also das partikelhaltige Wasser-Speichel-Gemisch aus dem Strom abgeschieden.

Die Prallflächen umfassen einerseits stegartige Leitvorsprünge 262 an der äußeren Mantelfläche 263 des Luftleiteinsatzes 260, die den schneckenförmigen Entwässerungsraum 221 nach außen hin begrenzt. Die Leitvorsprünge 262 haben die Gestalt von leicht aus der Vertikalen geneigten und leicht konvex gekrümmten Stegen, wobei die Neigung mit Bezug auf die Strömungsrichtung so ist, dass die Stege unten etwas nach hinten geneigt sind, und die Krümmung mit Bezug auf die Strömungsrichtung konvex ist.

Ferner umfassen die Prallvorsprünge einen Kranz an radialen Blättchen 264, die sich von der inneren Mantelfläche 265 des Luftleiteinsatzes 260, die den schneckenförmigen Entwässerungsraum 221 nach innen hin begrenzt, bis zur äußeren Mantelfläche 263 erstrecken und den Entwässerungsraum 221 nach unten hin begrenzen. Die Blättchen 264 sind gleichmäßig und alle auf derselben Höhe über den Umfang der inneren Mantelfläche 265 bzw. über die Erstreckung des schneckenförmigen Entwässerungsraums 221 verteilt. Die Blättchen 264 sind im spitzen Winkel von etwa 20°-30° so aus der Horizontalen geneigt, dass sie leicht angestellte Prallkonturen für den Luftstrom im Entwässerungsraum 221 bilden. Da die Blättchen 264 zudem in geringem Abstand zueinanderstehen, wobei die Vorderkante jedes Blättchens 264 die Hinterkante des darauffolgenden Blättchens 264 endet, ergibt sich eine treppenförmige Kontur.

Die unteren Enden der Leitvorsprünge 262 stehen an der Oberfläche eines korrespondierenden Blättchens 264 an, wobei sich an den Berührungspunkt ein zwischen dem Blättchen 264 und dem äußeren Mantel 263 ausgebildeter Spalt 266 anschließt, durch den am Leitvorsprung 262 abgeschiedenes Wasser nach unten aus dem Entwässerungsraum 221 abrinnen kann. Das Wasser wird durch die Kontur der Leitvorsprünge 262 in diesen Spalt 266 hineingeleitet.

Auch durch die Zwischenräume zwischen den Blättchen 264 bzw. Treppenstufen kann Wasser aus dem Entwässerungsraum 221 nach unten austreten.

Der entwässerte Luftstrom wird am Ende des Entwässerungsraums 221 durch einen Auslassstutzen 228 geleitet, wobei im Auslassstutzen 228 eine Verengung angeordnet ist, an der die austretende Luft aufgrund des Venturi-Effekts einen Unterdruck erzeugt. In diesen Unterdruckbereich mündet ein in den unteren Gehäuseteil eintauchendes Saugrohr 229 zum Ansaugen von gereinigtem Wasser. Dieser Vorgang wird später noch beschrieben.

Die im Entwässerungsraum 221 aus dem Luft-Flüssigkeits-Gemisch abgetrennte partikelhaltige Flüssigkeit fließt an einer leicht geneigten Bahn 267 am Boden 268 des oberen Gehäuseteils 220 durch eine Öffnung 269 in ein zentral im unteren Gehäuseteil 210 angeordnetes Fallrohr 211 bis in eine erste Sedimentationszone 212 am Boden des unteren Gehäuseteils 210.

Ausgehend von dieser ersten Sedimentationszone 212 steigt die Flüssigkeit, angetrieben durch die im Fallrohr 211 nachkommende Flüssigkeit, in einer ringförmigen Steigzone 213, die nach außen hin durch den Mantel des unteren Gehäuseteils 210 und nach innen hin durch das Fallrohr 211 begrenzt ist, nach oben. In der Steigzone 213 ist ein Siebboden 251 eingesetzt, welchen die Flüssigkeit auf ihrem Weg nach oben durchläuft. Auf dem Siebboden 251 sind verschiedene Schüttungen aufgebracht, die in den Figuren nicht dargestellt sind. Einzelne Schüttungen können jeweils auch über Böden voneinander getrennt sein. Das Wasser steigt mit den Partikeln in diese Schüttung bzw. Schüttungen hinein wobei die schwereren Partikel bereits in der ersten Sedimentationszone 212 absedimentieren und die Schwebepartikel in den Schüttungen verbleiben.

Zu den Schüttungen ist anzugeben, dass sie aus unterschiedlichen Materialien wie Aktivkohle, Zinkspänen, grobporiger Zeolith oder anderen Materialien bestehen können, wobei hier die jeweiligen Schichten jeweils verschiedene Korngrößen aufweisen. Dabei kann bevorzugt sein, ein gröberes Korn in den unteren Schichten vorzusehen und nach oben hin immer feinere Körner vorzusehen, um eine Klassierung der abgeschiedenen Partikel zu erreichen und in der feinsten Schicht ganz oben die kleinsten Schwebepartikel auffängt.

Das Wasser steigt dann weiter hoch und wird aufgrund des in dem als Luftstrahlpumpe ausgebildeten Auslassstutzens 228 herrschenden Unterdrucks durch das Saugrohr 229 in den Auslassstutzen 228 hineingezogen und verlässt gemeinsam mit der entwässerten Luft den Abscheider 200.

Von besonderer Bedeutung sind die kaskadenförmige Treppenstruktur, welche im Entwässerungsraum 221 durch die Blättchen 264 gebildet wird, sowie die Leitvorsprünge 262.

Durch die gezeigte Konstruktion des Abscheiders 200 gemäß dieser Ausführungsvariante wird in sehr vorteilhafter Weise erreicht, dass das Wasser durch das langsame Aufsteigen in die verschiedenen Schichten eine Ruhezone durchläuft und nicht in der Apparatur verwirbelt wird. Erst nach Durchlaufen der verschiedenen Schichten wird es dann an der Oberfläche durch das Saugrohr 229 abgesaugt. Darunter ist das Wasser aber in Ruhe, sodass die Sedimentation bzw. das Auffangen der Schwebepartikel hier optimal durchgeführt werden kann. Ein Mitreißen von Schwebepartikeln auch sehr kleiner Korngröße wird effektiv vermieden.

Das Gehäuse ist aus einem Kunststoff gefertigt und in mehreren Teilen einfach zusammengesteckt. So ist der Luftleiteinsatz 260 als separates Spritzgussteil ausgeführt und in einfacher Weise in den oberen Gehäuseteil 220 eingesteckt.

Wenn sich der Abscheider 200 im Einsatz mit Schwebepartikeln füllt, die üblicherweise aus Amalgam und sonstigen Stoffen aus der Zahnarztbehandlung bestehen, kann bei Erreichen der maximalen Kapazität das gesamte Gehäuse mit speziellen Schraubverschlüssen verschlossen und als Ganzes zur weiteren Aufarbeitung transportiert werden.

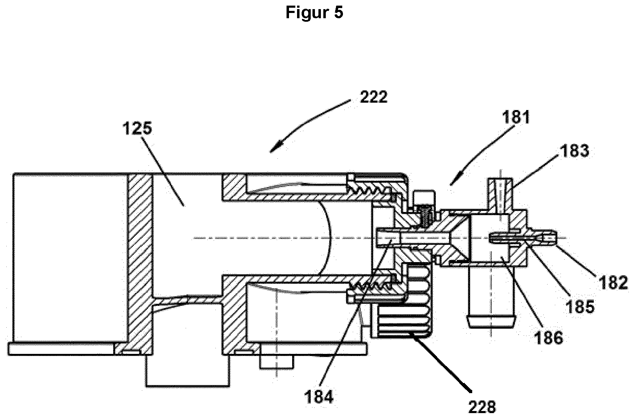

Eine Besonderheit des vorliegenden Dentalabscheiders liegt in der Ausbildung des Einlassstutzens 222, wie sie in Figur 5 zu erkennen ist.

Dieser umfasst eine Strahlpumpe 181 mit einem Treibmedienanschluss 182 für ein Treibmedium, einem Saugmedienanschluss 183 für ein Saugmedium und einen Austritt 184, der tangential in die Strömungsbahn des Entwässerungsbereichs 221 mündet. Kernstück der Strahlpumpe 181 bildet eine Venturi-Düse 185, durch die unter Druck stehende Luft getrieben wird, um in einer Mischkammer 186 einen Unterdruck zu erzeugen. Aufgrund dieses Unterdrucks wird durch den Saugmedienanschluss 182, der in die Mischkammer 186 mündet, ein Luft-Speichel-Gemisch von einem Speichelsauger angesaugt und mit der Druckluft aus dem Treibmedienanschluss 182 vermischt.

Das resultierende Luft-Speichel-Gemisch strömt mit hoher Geschwindigkeit durch den Austritt 184 in die Strömungsbahn und nimmt dort den bereits zuvor beschriebenen Verlauf. Die im Ausführungsbeispiel beschriebene Gestaltung der Prallkonturen ist insbesondere bei den hohen Mediengeschwindigkeiten, die sich aus der erfindungsgemäßen Ausbildung des Einlassstutzens 222 und aus der Verwendung von Druckluft als Triebmedium ergeben, besonders vorteilhaft, da sie eine sehr gute Luftführung auch bei hohen Mediengeschwindigkeiten erlaubt.

## Patentansprüche

1. Dentalabscheider (200) mit einem Gehäuse, in dessen oberen Bereich eine Entwässerungszone (221) zur Abtrennung von partikelhaltiger Flüssigkeit aus einem mit der Flüssigkeit beladenen Luftstrom ausgebildet ist, wobei das Gehäuse einen Einlass (222) für den beladenen Luftstrom aufweist, der tangential in die Entwässerungszone (221) mündet, und im Gehäuse unterhalb der Entwässerungszone (221) eine Abscheidezone zur Abtrennung der Partikel aus der partikelhaltigen Flüssigkeit ausgebildet ist, die von der Entwässerungszone (221) in die Abscheidezone sinkt,
**dadurch gekennzeichnet,**
**dass** die Entwässerungszone (221) eine gekrümmte Strömungsbahn umfasst und der Einlass (222) eine Strahlpumpe (181) mit einem Treibmedienanschluss (182) für ein Treibmedium, einem Saugmedienanschluss (183) für ein Saugmedium und einen Austritt (184) aufweist, wobei der Austritt (184) tangential in die Strömungsbahn mündet.

2. Dentalabscheider (200) nach Anspruch 1, **dadurch gekennzeichnet, dass** die Entwässerungszone (221) eine gekrümmte Strömungsbahn umfasst, die an ihrer Unterseite durch einen Kranz an Prallblättchen (264) begrenzt ist.

3. Dentalabscheider (200) nach Anspruch 2, **dadurch gekennzeichnet**, die Prallblättchen (264) gleichmäßig und/oder alle auf derselben Höhe über die Erstreckung des Strömungskanals verteilt sind und/oder dass die Prallblättchen (264) im spitzen Winkel von vorzugsweise 10°-40° und weiter vorzugsweise 20°-30° in Strömungsrichtung nach oben aus der Horizontalen geneigt sind.

4. Dentalabscheider (200) nach Anspruch 2 oder 3, **dadurch gekennzeichnet, dass** der in der Draufsicht erkennbare Abstand zwischen den Vorderkanten und Hinterkanten aufeinanderfolgender Prallblättchen (264) kleiner als die halbe umfängliche Erstreckung und vorzugsweise kleiner als ein Viertel der umfänglichen Erstreckung der Prallblättchen (264) ist.

5. Dentalabscheider (200) nach einem der Ansprüche 2 bis 4, **dadurch gekennzeichnet, dass** die Prallblättchen (264) radial von einer gekrümmten Wand innerhalb des Gehäuses abstehen, welche die Innenseite der Strömungsbahn definiert.

6. Dentalabscheider (200) nach einem der Ansprüche 2 bis 5, **dadurch gekennzeichnet, dass** an einer Wand (263), welche die äußere Begrenzung des Strömungskanals bildet, stegartige Leitvorsprünge (262) vorgesehen sind, die in den Strömungskanal ragen, wobei vorzugsweise vorgesehen ist, dass die unteren Enden der Leitvorsprünge (262) an der Oberseite eines korrespondierenden Prallblättchens (264) anstehen, wobei weiter vorzugsweise vorgesehen ist, dass zwischen der Außenkante der Prallblättchen (264) und der Innenoberfläche der Wand, welche die äußere Begrenzung des Strömungskanals bildet, jeweils ein Spalt (266) ausgebildet ist, der sich in Abflussrichtung auf der Oberseite des Prallblättchens (264) an den Berührungspunkt anschließt.

7. Dentalabscheider (200) nach einem der Ansprüche 2 bis 6, **dadurch gekennzeichnet, dass** die Begrenzungswände des Strömungskanals nebst Prallblättchen (264) an einem Einsatz ausgebildet sind, der im Gehäuse und vorzugsweise im oberen Gehäuseteil (220) aufgenommen ist.

8. Dentalabscheider (200) nach einem der Ansprüche 2 bis 7, **dadurch gekennzeichnet, dass** unterhalb des Kranzes an Prallblättchen (264) eine Auffangzone vorgesehen ist, um das an den Prallkonturen aus dem Luftstrom abgeschiedene Wasser aufzufangen.

9. Dentalabscheider (200) nach einem der vorhergehenden Ansprüche, **dadurch gekennzeichnet, dass** im Bodenbereich des Gehäuses eine Sedimentationszone (212) ausgebildet ist, die nach oben hin durch einen Filter (251) von einem Absaugbereich abgegrenzt ist.

10. Dentalabscheider (200) nach einem der vorhergehenden Ansprüche, **dadurch gekennzeichnet, dass** das Gehäuse ferner einen Auslass (228) für den behandelten Luftstrom aufweist, der am distalen Ende der gekrümmten Strömungsbahn von der Entwässerungszone (221) nach außen führt.

11. Dentalabscheider (200) nach den Ansprüchen 9 und 10, **dadurch gekennzeichnet, dass** innerhalb des Auslasses (228) eine Venturi-Düse (185) mit einer Verengung ausgebildet ist, in welche ein aus dem Absaugbereich der Abscheidezone führendes Saugrohr (229) mündet.

12. Zahnärztliche Behandlungseinheit umfassend einen Speichelsauger, eine Druckquelle und einen Dentalabscheider (200) nach einem der vorhergehenden Ansprüche, wobei der Speichelsauger mit dem Saugmedienanschluss (183) und eine Druckquelle mit dem Treibmedienanschluss (182) verbunden sind.

13. Zahnärztliche Behandlungseinheit nach Anspruch 12, **dadurch gekennzeichnet, dass** es sich bei der Druckquelle um eine unter Druck stehende Gasflasche handelt, die vorzugsweise mit Druckluft befüllt ist, wobei vorzugsweise ein steuerbares Ventil zwischen der Gasflasche und der Strahlpumpe (181) angeordnet ist, um die Strahlpumpe (181) selektiv aktivieren zu können.

14. Zahnärztliche Behandlungseinheit nach Anspruch 13, **dadurch gekennzeichnet, dass** ein steuerbares Ventil zwischen der Gasflasche und der Strahlpumpe (181) angeordnet ist.

15. Zahnärztliche Behandlungseinheit nach Anspruch 13, **dadurch gekennzeichnet, dass** es sich bei der Druckquelle um eine Pumpe handelt, vorzugsweise um eine Luftpumpe.

## Claims

1. Dental evacuator (200) having a housing, in the upper region of which a drainage zone (221) is provided for separating particle-containing liquid from an air stream which is loaded with the liquid, wherein the housing has an inlet (222) for the loaded air stream which opens tangentially into the drainage zone (221), and in the housing below the drainage zone (221) an evacuation zone is provided for separating the particles from the particle-containing liquid falling from the drainage zone (221) into the evacuation zone,
**characterised in that**
the drainage zone (221) comprises a curved flow path, and the inlet (222) has a jet pump (181) with a propellant connection (182) for a propellant, a suction medium connection (183) for a suction medium and an outlet (184), wherein the outlet (184) opens tangentially into the flow path.

2. Dental evacuator (200) according to claim 1, **characterised in that** the drainage zone (221) comprises a curved flow path, which is limited on its lower side by a ring of baffle blades (264).

3. Dental evacuator (200) according to claim 2, **characterised in that** the baffle blades (264) are distributed uniformly and/or all at the same height over the extent of the flow channel and/or **in that** the baffle blades (264) are inclined at an acute angle of preferably 10°-40° and more preferably 20°-30° upwards from the horizontal in the direction of flow.

4. Dental evacuator (200) according to claim 2 or 3, **characterised in that** the distance between the leading edges and trailing edges of successive baffle blades (264) which is visible in the top view is less than half the circumferential extent and preferably less than a quarter of the circumferential extent of the baffle blades (264).

5. Dental evacuator (200) according to any one of claims 2 to 4, **characterised in that** the baffle blades (264) project radially from a curved wall within the housing defining the inside of the flow path.

6. Dental evacuator (200) according to any one of claims 2 to 5, **characterised in that** web-like guide projections (262) are provided on a wall (263), which form the outer boundary of the flow channel and project into the flow channel, wherein it is preferably provided that the lower ends of the guide projections (262) abut the upper side of a corresponding baffle blade (264), wherein it is more preferably provided that a gap (266) is formed between the outer edge of the baffle blades (264) and the inner surface of the wall forming the outer boundary of the flow channel, respectively, which gap adjoins the point of contact on the upper side of the baffle blade (264) in the discharge direction.

7. Dental evacuator (200) according to any one of claims 2 to 6, **characterised in that** the boundary walls of the flow channel together with baffle blades (264) are formed on an insert, which is accommodated in the housing and preferably in the upper housing part (220).

8. Dental evacuator (200) according to any one of claims 2 to 7, **characterised in that** a collection zone is provided below the ring of baffle blades (264) in order to collect the water separated from the air stream at the baffle contours.

9. Dental evacuator (200) according to any one of the preceding claims, **characterised in that** a sedimentation zone (212) is formed in the base region of the housing, which is separated from an extraction zone at the top by a filter (251).

10. Dental evacuator (200) according to any one of the preceding claims, **characterised in that** the housing further has an outlet (228) for the treated air stream, which runs outwards from the drainage zone (221) at the distal end of the curved flow path.

11. Dental evacuator (200) according to claims 9 and 10, **characterised in that** a venturi nozzle (185) with a constriction is formed inside the outlet (228), into which a suction pipe (229) leading out of the suction area of the separation zone opens.

12. Dental treatment unit comprising a saliva ejector, a pressure source and a dental evacuator (200) according to any one of the preceding claims, wherein the saliva ejector is connected to the suction medium connection (183) and a pressure source is connected to the propellant connection (182).

13. Dental treatment unit according to claim 12, **characterised in that** the pressure source is a pressurised gas bottle, preferably filled with compressed air, wherein preferably a controllable valve is arranged between the gas bottle and the jet pump (181) in order to be able to selectively activate the jet pump (181).

14. Dental treatment unit according to claim 13, **characterised in that** a controllable valve is arranged between the gas bottle and the jet pump (181).

15. Dental treatment unit according to claim 13, **characterised in that** the pressure source is a pump, preferably an air pump.

## Revendications

1. Séparateur dentaire (200) avec un boîtier, dans la région supérieure duquel est réalisée une zone de déshydratation (221) pour séparer un liquide contenant des particules d'un courant d'air chargé du liquide, le boîtier présentant une entrée (222) pour le courant d'air chargé, qui débouche tangentiellement dans la zone de déshydratation (221), et une zone de séparation étant réalisée dans le boîtier sous la zone de déshydratation (221) pour séparer les particules du liquide contenant des particules qui descend de la zone de déshydratation (221) dans la zone de séparation,
**caractérisé en ce que**
la zone de déshydratation (221) comprend une voie d'écoulement incurvée et l'entrée (222) présente une pompe à jet (181) avec un raccord de milieu de propulsion (182) pour un milieu de propulsion, un raccord de milieu d'aspiration (183) pour un milieu d'aspiration et une sortie (184), la sortie (184) débouchant tangentiellement dans la voie d'écoulement.

2. Séparateur dentaire (200) selon la revendication 1, **caractérisé en ce que** la zone de déshydratation (221) comprend une voie d'écoulement incurvée qui est délimitée sur son côté inférieur par une couronne de lamelles déflectrices (264).

3. Séparateur dentaire (200) selon la revendication 2, **caractérisé en ce que** les lamelles déflectrices (264) sont réparties uniformément et/ou toutes à la même hauteur sur l'étendue du canal d'écoulement et/ou **en ce que** les lamelles déflectrices (264) sont inclinées vers le haut par rapport à l'horizontale selon un angle aigu de préférence de 10° à 40° et encore de préférence de 20° à 30° dans la direction d'écoulement.

4. Séparateur dentaire (200) selon la revendication 2 ou 3, **caractérisé en ce que** la distance perceptible en vue de dessus entre les bords avant et les bords arrière de lamelles déflectrices successives (264) est inférieure à la moitié de l'étendue circonférentielle et de préférence inférieure à un quart de l'étendue circonférentielle des lamelles déflectrices (264).

5. Séparateur dentaire (200) selon l'une quelconque des revendications 2 à 4, **caractérisé en ce que** les lamelles déflectrices (264) partent radialement à l'intérieur du boîtier à partir d'une paroi incurvée, qui définit le côté intérieur de la voie d'écoulement.

6. Séparateur dentaire (200) selon l'une quelconque des revendications 2 à 5, **caractérisé en ce que** des protubérances de guidage (262) de type nervures qui font saillie dans le canal d'écoulement sont prévues sur une paroi (263) qui forme la délimitation extérieure du canal d'écoulement, il étant de préférence prévu que les extrémités inférieures des protubérances de guidage (262) reposent sur le côté supérieur d'une lamelle déflectrice (264) correspondante, il étant encore de préférence prévu qu'entre le bord extérieur de la lamelle déflectrice (264) et la surface intérieure de la paroi qui forme la délimitation extérieure du canal d'écoulement, il est réalisé respectivement une fente (266) qui se raccorde au point de contact dans la direction du flux sortant sur le côté supérieur de la lamelle déflectrice (264).

7. Séparateur dentaire (200) selon l'une quelconque des revendications 2 à 6, **caractérisé en ce que** les parois de délimitation du canal d'écoulement ainsi que les lamelles déflectrices (264) sont réalisées sur un insert qui est logé dans le boîtier et de préférence dans la partie de boîtier supérieure (220).

8. Séparateur dentaire (200) selon l'une quelconque des revendications 2 à 7, **caractérisé en ce qu'**une zone de collecte est prévue sous la couronne de lamelles déflectrices (264) pour collecter l'eau séparée du courant d'air au niveau des contours déflecteurs.

9. Séparateur dentaire (200) selon l'une quelconque des revendications précédentes, **caractérisé en ce qu'**une zone de sédimentation (212) est réalisée dans la région de fond du boîtier, qui est délimitée vers le haut par un filtre (251) par rapport à une région d'aspiration.

10. Séparateur dentaire (200) selon l'une quelconque des revendications précédentes, **caractérisé en ce que** le boîtier présente en outre une sortie (228) pour le courant d'air traité, qui, à l'extrémité distale de la voie d'écoulement incurvée, mène de la zone de déshydratation (221) vers l'extérieur.

11. Séparateur dentaire (200) selon les revendications 9 et 10, **caractérisé en ce qu'**à l'intérieur de la sortie (228) est réalisée une buse Venturi (185) avec un rétrécissement dans lequel débouche un tube d'aspiration (229) menant hors de la région d'aspiration de la zone de séparation.

12. Unité de traitement dentaire comprenant un aspirateur de salive, une source de pression et un séparateur dentaire (200) selon l'une quelconque des revendications précédentes, l'aspirateur de salive étant relié au raccord de milieu d'aspiration (183) et une source de pression étant reliée au raccord de milieu de propulsion (182).

13. Unité de traitement dentaire selon la revendication 12, **caractérisée en ce que** la source de pression consiste en une bouteille de gaz sous pression, de préférence remplie d'air comprimé, une soupape commandable étant de préférence agencée entre la bouteille de gaz et la pompe à jet (181) afin de pouvoir activer sélectivement la pompe à jet (181).

14. Unité de traitement dentaire selon la revendication 13, **caractérisée en ce qu'**une soupape commandable est agencée entre la bouteille de gaz et la pompe à jet (181).

15. Unité de traitement dentaire selon la revendication 13, **caractérisée en ce que** la source de pression consiste en une pompe, de préférence une pompe à air.
